# EUROPEAN PATENT APPLICATION

(11) **EP 4 296 281 A1**
(43) Date of publication of application: **27.12.2023**
(21) Application number: 22180805.8
(22) Date of filing: 23.06.2022
(51) Int. Cl.: C07K 16/28, C07K 16/18, A61K 39/00, C07K 14/725, C07K 14/705, A61P 35/00

(54) **TARGETING MODULES AGAINST CD123 AND A TAG FOR USE IN A METHOD FOR STIMULATING A UNIVERSAL CHIMERIC ANTIGEN RECEPTOR-MEDIATED IMMUNE RESPONSE IN A MAMMAL**

(71) Applicant: AvenCell Therapeutics Inc., Cambridge, MA 02138 (US)
(72) Inventor: Spehr, Johannes, 01307 Dresden (DE); Ehninger, Armin, 01307 Dresden (DE)
(74) Representative: Kailuweit & Uhlemann Patentanwälte Partnerschaft mbB

(57) **Abstract**

The present invention relates to a targeting module comprising at least one CD123-binding domain and a tag-binding domain binding the human La epitope E5B9, a nucleic acid, a vector or a cell comprising a nucleotide sequence encoding the targeting module, a pharmaceutical composition and a kit comprising the targeting module and a vector or a cell comprising a nucleotide sequence encoding a reversible chimeric antigen receptor.

## Description

The present invention relates to a targeting module comprising at least one CD123-binding domain and a tag-binding domain binding the human La epitope E5B9, a nucleic acid, a vector or a cell comprising a nucleotide sequence encoding the targeting module, a pharmaceutical composition and a kit comprising the targeting module and a vector or a cell comprising a nucleotide sequence encoding a reversible chimeric antigen receptor.

Chimeric antigen receptors (CARs) are artificial receptors consisting of a binding moiety, which provides the antigen-specificity and one or several signaling chains derived from immune receptors (Cartellieri *et al.* 2010). Immune cells, genetically modified to express CARs, can be used to bind cells or tissue structures expressing the appropriate target of the CAR binding moiety. Cross-linking leads to an induction of signal pathways via the CAR signaling chains, which will change the biologic properties of the CAR-engrafted immune cell. In contrast, CAR activation in gene-modified regulatory T cells (Tregs) leads to an activation of Treg-specific immunomodulatory and suppressive mechanisms like interleukin (IL)-10 or tumor growth factor-beta (TGF-β) secretion. The adoptive transfer of immune cells engineered with chimeric antigen receptors (CARs) is currently considered a highly promising therapeutic option for the treatment of otherwise incurable malignant, infectious or autoimmune diseases.

However, the conventional CAR technology comes along with a number of critical issues, which need to be solved before this treatment modality can be widely applied for clinical treatments. First of all, several safety issues have to be addressed. So far, immune responses of T cells engineered with conventional CARs are difficult to control after infusion into the patient. Serious adverse event rates are high (Titov *et al.* 2018). Especially unexpected target gene expression on normal tissue may provoke a rapid and rigorous immune reaction of engineered T cells against normal cells, which can cause severe side effects (Morgan *et al.* 2010). Moreover, as CAR-T cells are a new class of self-amplifying cell drugs, infused T cells can undergo a vigorous expansion in the presence of heavy tumor burden leading to tumor lysis syndrome, cytokine release syndrome and macrophage activation syndrome (Brudno and Kochenderfer 2016). Another drawback of conventional CAR technology is the restriction of engineered T cell retargeting to a single antigen. Such a monotherapeutic approach implies the risk for the development of tumor escape variants, which have lost the target antigen during treatment. The emergence of tumor escape variants under conventional CAR T cell therapy after several months was already observed in clinical trials (Sotillo *et al.* 2015). Taken together, these obstacles restrict the application of CAR T cells to very few indications. In fact, examples of clinical effectiveness have been restricted to CD19- and BCMA-targeting CAR T cells until now.

Modular switchable "universal" CAR T (UniCAR) approaches can overcome these limitations by separating antigen recognition and activating domain of a CAR into two separate operational units. T cells are engineered to express a CAR with a universal binding domain recognizing a tag (Cartellieri *et al.* 2016). Antigen-specificity is provided by soluble adapter proteins, which consist of an antigen-binding domain fused to the tag recognized by the universal CAR. Cartellieri *et al.* describe the treatment of CD33- and/or CD123-positive acute myeloid leukemia cells *in vitro* and *in vivo.*

Next to the UniCAR approach for recognizing various antigens (EP 2 990 416 A1) a reversed universal CAR (RevCAR) approach is known that promotes binding of an immune cell engineered to express a RevCAR comprising a tag to a target cell through an adaptor molecule comprising a tag-binding domain and a target cell-binding domain (EP 3 581 200 A1).

The object of the present invention is to provide an improved targeting module for use in a RevCAR system.

The object has been solved by a targeting module comprising at least one CD123-binding domain and a tag-binding domain that binds to a human La epitope E5B9 comprising a V_{L}-linker-V_{H} structure according to the present invention.

Advantageously, the targeting module according to the invention comprises
i) at least one CD123-binding domain comprising a sequence with at least 95 % identity, preferably 99 % identity, with the sequence according to SEQ ID No. 22 and SEQ ID No. 23, and
ii) a tag-binding domain binding a human La epitope E5B9 comprising a V_{L}-linker-V_{H} structure, wherein the V_{L} region of the tag-binding domain comprises a sequence with at least 95 % identity, preferably 99 % identity, with the sequence according to SEQ ID No. 19 and/or the V_{H} region of the tag-binding domain comprises a sequence with at least 95 % identity, preferably 99 % identity, with the sequence according to SEQ ID No. 20.

As used herein, the term "targeting module" refers to a molecule, preferably a polypeptide or protein with at least two different domains, wherein each domain is specific for a target or a uniform group of targets, respectively, wherein at least one domain is specific for a target cell, in particular the CD123-binding domain; and one domain is specific for a reversible chimeric antigen receptor, in particular the tag-binding domain. In embodiments, the targeting module is isolated. Preferably, the targeting module according to the invention is expressed as a recombinant protein. In further embodiments, the targeting module is chemically synthesized.

The term "autoimmune disorder" refers to an abnormal immune response of the body against substances and tissues normally present in the body (autoimmunity).

As used herein, the term "domain" refers to a part of a protein sequence, which can exist and function independently from the rest of the protein.

As used herein, the term "specific" refers to the ability of an antibody or antibody fragment or a protein, peptide or low molecular weight organic ligand to recognize and bind with a binding partner (e.g. a tumor antigen) protein present in a sample, but not substantially recognize or bind other molecules in the sample.

As used herein, the term "binds" or "binding" refers to a non-covalent binding, in particular ionic bonds, hydrogen bonds, Van der Waals forces and/or hydrophobic interactions.

As used herein, the term "mutants" refers to peptides or proteins having at least 90 % sequence identity to the named antibodies, antibody fragments, proteins or peptides, preferably at least 95 % sequence identity. Advantageously, the mutants are capable of having one or more activities of the named antibodies, antibody fragments, peptides or proteins.

In embodiments, mutants are truncated versions of peptides or proteins. As used herein, the term "truncated versions" refers to shortened peptides or proteins having at least 90 % sequence identity to the named peptides or proteins, preferably at least 95 % sequence identity, more preferably having a chain length of at least 90 % and a sequence identity of 100 %, most preferably a chain length of at least 95 % and a sequence identity of 100 %. Advantageously, the truncated version has at least 80 %, preferably of at least 90 %, more preferably of at least 95 %; of the activity of the named peptide or protein.

As used herein, the term "nuclear protein" refers to a protein found in the cell nucleus.

Advantageously, tags, which are peptide sequences from nuclear antigens, cannot be accessed and bound by the corresponding tag-binding domain in the context of the native protein under physiological conditions. Further advantageously, the tag is not immunogenic. This leads to minimization of the risk of uncontrolled on-target off-site toxicities by CAR-expressing immune cells like the release of toxic levels of cytokines, referred to variously as cytokine storms or cytokine release syndrome (CRS).

As used herein, the term "administered in combination" refers to a treatment, wherein the targeting module is administered prior to, simultaneously with and/or after the administration of the vector or cell comprising a nucleotide sequence encoding a reversible chimeric antigen receptor (RevCAR).

As used herein, the term "antibody" refers to a protein, which binds antigens via the antigen-binding fragment variable region (Fab). This is composed of one constant and one variable domain of each of the heavy (V_{H}) and the light chain (V_{L}). As used herein, the term "antibody fragment or antigen-binding fragment" refers to a protein comprising at least the V_{L} or V_{H} of an antibody. In embodiments, antibody fragments are selected from single-chain variable fragments (scFv), single-chain antibodies, F(ab')2 fragments, Fab fragments, and fragments produced by a Fab expression library or single-domain antibodies (nanobodies).

As used herein, the term "single-chain variable fragment (scFv)" refers to an artificial antibody fragment comprising a variable domain of a light chain and a variable domain of a heavy chain of an antibody covalently linked. In embodiments, the V_{L} and V_{H} of an antibody are covalently linked by a short peptide of 10 to 25 amino acids. In further embodiments, the short peptide links the N-terminus of the V_{H} with the C-terminus of the V_{L}, or vice versa.

As used herein, the term "CDR (Complementarity-determining regions)" refers to parts of the variable chains in antibodies or antibody fragments, where the antibodies or antibody fragments bind to their specific antigen. An antibody comprises three CDRs (CDR1, CDR2 and CDR3), arranged non-consecutively, on the amino acid sequence of each variable domain and thus, six CDRs on the two variable domains (V_{H} and V_{L}), which can come into contact with the antigen.

In embodiments, V_{L} and V_{H} are connected via a glycine-serine linker with the structure (GₓS_{y}) with x and y selected from 1 to 10, preferably 3 to 5. Mostly preferred are 1 to 10 repeats of the sequence G₄S₁ (SEQ ID No. 24). Moreover, linkers are preferred that are constituted of a peptide sequence that can increase the protease resistance of the antibody derivatives.

In embodiments of the invention, the linker of the tag-binding domain comprises 20 to 30 amino acids, preferably 25 amino acids.

In further embodiments, the linker of the tag-binding domain comprises a linker according SEQ ID No. 25 or SEQ ID No. 26.

In embodiments, the antibody is obtained from an animal species, preferably from a mammal such as human, simian, mouse, rat, rabbit, guinea pig, horse, cow, sheep, goat, pig, dog or cat. Preferably, the antibody or antibody fragment is a human, humanized or deimmunized antibody. Humanized antibodies can be prepared in various ways, for example, by resurfacing and CDR grafting. In case of resurfacing, a combination of molecular modeling, statistical analyses, and mutagenesis is used to modify all non-CDR regions on the surface of the antibody to become similar to the surface of antibodies of the target organism. In CDR grafting, the CDR regions according to the invention are introduced into known human framework regions, which are similar in sequence to the original ones. Deimmunized antibodies can be obtained by specifically mutating residues that confer immunogenicity hotspots as predicted based on in silico peptide-MHC affinity prediction.

In embodiments, the antibody or antibody fragment is a polyclonal, a monoclonal or a chimeric antibody, wherein an antigen-binding region of a non-human antibody is transferred into the framework of a human antibody by recombinant DNA techniques including in silico design.

In embodiments, antibodies to a selected tag or antigen may be produced by immunization of various hosts including, but not limited to, goats, rabbits, rats, mice, humans, through injection with cells expressing a particular protein, DNA or RNA encoding for the protein, the protein itself or any portion, fragment or oligopeptide that retain immunogenic properties of the protein.

In preferred embodiments, the CD123-binding domain is an antibody or antigen binding fragment.

In embodiments, the CD123-binding domain comprises the sequences SEQ ID No. 22 and SEQ ID No. 23.

In preferred embodiments, the CD123-binding domain comprises a sequence according to SEQ ID No. 27.

According to the invention, the tag-binding domain binds to a tag from the human La epitope E5B9. In embodiments, the tag-binding domain is an antibody or an antigen-binding fragment, preferably comprising a VL according to the following sequence:
DIVMTQSPDSLAVSLGERATINCX₂₄SSQSLLNSRTX₃₅KNYLAWYQQKPGQPPKLLIYWASTR X₆₁SGVPDRFSGSGSGTDFTLTISSLQAEDVAVYYCKQSYNLX₁₀₁TFGGGTKVEIK (SEQ ID No. 19), wherein X₂₄, X₃₅, X₆₁ and X₁₀₁ are independently from each other selected from a proteinogenic alpha-amino acid residue;
or a sequence having at least 90 % sequence identity, preferably at least 95 % sequence identity; to sequence SEQ ID No. 21.

In embodiments, the tag-binding domain constitutes an anti-La epitope scFv.

In some embodiments, X₂₄ to X₁₀₁ are selected as follows:
X₂₄ is selected from polar and/or positive charged residues, such as Serine, Threonine, Asparagine, Glutamine, Histidine, Lysine and Arginine; preferably Lysine or Arginine;
X₃₅ is preferably selected from Lysine and Proline;
X₆₁ is selected from polar and charged residues, such as Asparagine, Aspartic Acid, Glutamine, Glutamic acid, Histidine, Lysine and Arginine, preferably Glutamic acid and Lysine;
X₁₀₁ is selected from hydrophobic residues, such as Isoleucine, Leucine, Valine, Alanine, Methionine, Phenylalanine, Proline and Tryptophan; preferably Leucine or Proline.

Preferably, the tag-binding domain comprises a sequence having each at least 90 % sequence identity, preferably at least 95 % sequence identity; to the sequences according to SEQ ID NO. 20 (V_{H}) and SEQ ID NO. 21 (V_{L}).

Most preferably, the tag-binding domain constitutes the anti-La 5B9 scFv according to SEQ ID NO. 20 (V_{H}) and SEQ ID NO. 21 (V_{L}).

In embodiments, the length of the target module is in the range of 20 to 1600 amino acids, preferably 200 to 800 amino acids.

In embodiments, the targeting module comprises one of the sequences according to SEQ ID No.3 to SEQ ID No. 10.

In a further aspect, the invention provides a nucleic acid, a vector or a cell comprises a nucleotide sequence encoding a targeting module according to the invention.

In embodiments, the nucleic acid, vector or cell according comprises one of the sequences according to SEQ ID No. 11 to 18.

In a further aspect, the invention provides a pharmaceutical composition comprising the targeting module according to the invention and a pharmaceutically acceptable thinner or carrier.

The pharmaceutical composition is preferably administered parenterally, particularly preferred intravenously. In embodiments, the pharmaceutical composition is present in a form suitable for intravenous administration. Preferably, the pharmaceutical composition is a solution, emulsion or suspension.

In embodiments, the pharmaceutical composition is an injectable buffered solution comprising a concentration in the range of 1 ng/ml to 500 mg/ml of the targeting module, the nucleic acid, vector and/or cell according to the invention, preferably in the range of 50 µg/ml to 5 mg/ml.

The pharmaceutical composition comprises a pharmaceutically acceptable thinner (dilution agent) or carrier. In embodiments, the carrier is selected from water, an aqueous buffer solution, 0.9 % saline solution, 5 % glucose, 5 % xylitol, 0.3 % glycine solution, ringer solutions or amino acid solutions. In further embodiments, the aqueous buffer solution is selected from an aqueous histidine, sodium succinate, sodium citrate, sodium phosphate or potassium phosphate-buffered solution with a pH value in the range of pH 5.0 to pH 7.0. In embodiments, the aqueous buffer solution has a buffer concentration in the range of 1 mmol/l (mM) to 500 mM, preferably in the range of 5 mM to 20 mM, especially preferred in the range of 5 mM to 10 mM.

In embodiments, the carrier comprises sodium chloride, preferably in a concentration in the range of 1 mM to 300 mM, especially preferred 150 mM.

In embodiments, the pharmaceutical composition further comprises a stabilizer, preferably with a concentration in the range of 1 mM to 900 mM, especially preferred in the range of 50 mM and 600 mM. In embodiments, the stabilizer is sucrose, trehalose or L-methionine.

In some embodiments, the pharmaceutical composition further comprises pharmaceutically acceptable excipients. The term "pharmaceutically acceptable excipients" refers to compounds, which provide approximately physiological conditions and/or increase the stability, such as agents for adjusting the pH value and buffering agents, agents for adjusting the toxicity and the like. In embodiments, pharmaceutically acceptable excipients are selected from sodium acetate, sodium chloride, potassium chloride, calcium chloride, sodium lactate and polysorbate-80, preferably polysorbate-80 in the range of 0.0001 % (w/v) to 1 % (w/v), especially preferred in the range of 0.001 % (w/v) to 0.1 % (w/v).

In preferred embodiments, the pharmaceutical composition comprises the targeting module in a dosage quantity in the range of 25 µg/day to 100 mg/day, preferably dosage quantities in the range of 0.1 mg/day to 20 mg/day.

In further embodiments, the pharmaceutical composition is sterile. The pharmaceutical composition is sterilized by conventional well-known techniques including, but not limited to, sterile filtration.

In embodiments, the pharmaceutical composition is used for administration to a subject.

In embodiments, the pharmaceutical composition is lyophilized prior to storage or stored as solution at ambient temperature or below, including, but not limited to, frozen storage.

In embodiments, the pharmaceutical composition is reconstituted and/or diluted in an infusion and stabilizer solution prior to administration to a subject. The solutions used for reconstitution or infusion/stabilization may contain any of the components mentioned for the pharmaceutical composition or similar components.

In a further aspect, the invention provides the targeting module according to the invention, a nucleic acid, a vector or a cell comprising a nucleotide sequence encoding the targeting module according to invention or a pharmaceutical composition comprising the targeting module according to the invention and a pharmaceutically acceptable thinner or carrier for use in a method for stimulating a chimeric antigen receptor-mediated immune response in a mammal, preferably for use in the treatment of cancer, infectious disease or autoimmune disease.

In embodiments, the targeting module is administered in combination with a vector or a cell comprising a nucleotide sequence encoding a RevCAR,
wherein the RevCAR comprises
- a tag, wherein the tag is the human La epitope E5B9 (SEQ ID No. 28),
- an extracellular hinge and transmembrane domain and
- a signal transduction domain,
wherein the tag-binding domain of the targeting module binds to the tag of the RevCAR.

In embodiments, the pharmaceutical composition for use in a method for stimulating a chimeric antigen receptor-mediated immune response in a mammal according to the invention further comprises a vector or a cell comprising a nucleotide sequence encoding a RevCAR, wherein the RevCAR comprises
- a tag, wherein the tag is the human La epitope E5B9 (SEQ ID No. 28),,
- an extracellular hinge and transmembrane domain and
- a signal transduction domain,
wherein the tag-binding domain of the targeting module binds to the tag of the RevCAR.

In a further aspect, the invention provides a kit comprising
a) a targeting module or the nucleic acid, vector or cell according to the invention, and
b) a vector or a cell comprising a nucleotide sequence encoding a RevCAR,
wherein the RevCAR comprises
- a tag, wherein the tag is the human La epitope E5B9 (SEQ ID No. 28),,
- an extracellular hinge and transmembrane domain and
- a signal transduction domain,
wherein the tag-binding domain of the targeting module binds to the tag of the RevCAR.

In embodiments, the extracellular hinge and transmembrane domain of the RevCAR is selected from the group comprising a hinge and transmembrane domain of a human CD28 molecule, a CD8a chain NK cell receptor, parts of the constant region of an antibody and combinations thereof.

In further embodiments, the signal transduction domain of the RevCAR is selected from the group comprising cytoplasmic regions of CD28, CD137 (4-1BB), CD134 (OX40), CD278 (ICOS), DAP10 and CD27, programmed cell death-1 (PD-1), cytotoxic T-lymphocyte antigen 4 (CTLA-4), cytoplasmic regions of CD3 chains, DAP12, CD122 (interleukin-2 receptor β), CD132 (interleukin-2 receptor γ), CD127 (interleukin-7 receptor α), CD360 (interleukin-21 receptor), activating Fc receptors and mutants thereof.

In further embodiments, the kit according to the invention comprises at least one further targeting module or at least one further nucleic acid, vector or cell encoding a further targeting module, wherein the at least one further targeting module comprises at least one target cell-binding domain and a tag-binding domain,
wherein the at least one target cell-binding domain is an antibody, antibody fragment, a protein, a peptide or a low molecular weight organic ligand that binds to surface antigens selected from the group comprising CD2, CD3, CD4, CD5, CD7, CD8, CD10, CD15, CD19, CD20, CD22, CD23, CD25, CD30, CD33, CD38, CD44, CD44v6 CD52, CD66a, CD66b, CD66c, CD66d, CD66e, CD66f, CD90, CD99, CD123, CD133, CD135, CD150 CD181, CD182, CD184, CD223, CD229, CD269, CD273, CD274, CD276, CD279, CD319, CD366 and CD371, cytokine receptors, preferably interleukin receptors, especially preferred IL-8Rα, IL-8Rβ, IL-11Rα, IL-11Rβ, IL13Rα1; CXCR4, c-Met, mesothelin, members of the epidermal growth factor receptor family and mutants thereof, especially preferred ErbB1, ErbB2, ErbB3, ErbB4 or mutants thereof; members of the tumor necrosis factor receptor superfamily, claudins, ephrins, ephrin receptors, especially preferred EphA1-10, EphA5 or EphB1-6; fucosyl transferases, prostate specific antigens, preferably PSCA and PSMA; embryonic antigens, preferably CEA and fetal acethylcholine receptor; members of the vascular endothelia growth factor family, EpCAM, AFP, members of the intercellular adhesion molecule family, C-type lectins, integrins, members of the mucin protein family, FSHR, HMW-MAA, FBP, folate receptors, somatostatin receptors, ligands of the NKG2D receptor, members of the epithelia glycoprotein family, diasialogangliosides, glypicans, G protein-coupled receptors, human papillomavirus proteins, cancer-testis antigens, fibroblast activation proteins, members of the carbonic anhydrase family, members of the carbohydrate antigen family, Notch ligands, MCSP, glycoprotein A33, guanylate cyclase 2C and tumor-specific glycans, including mutants and analogues of the named antibodies, antibody fragments, proteins, peptides or low molecular weight organic ligands,
wherein the targeting module and the at least one further targeting module comprise identical tag-binding domains.

In further embodiments, the kit according to the invention comprises the targeting module and/or the vector or cell comprising a nucleotide sequence encoding a RevCAR in the form of a pharmaceutical composition.

In further embodiments, the kit according to the invention is used in a method for stimulating a chimeric antigen receptor-mediated immune response in a mammal, preferably for use in the treatment of cancer, infectious disease or autoimmune disease.

In embodiments, the targeting module is administered on its own, preferably one hour to 2 days, more preferably 4 to 24 hours, prior to the administration of the vector or cell comprising a nucleotide sequence encoding a RevCAR. Advantageously, the administration of the targeting module prior to the administration of the vector or cell comprising a nucleotide sequence encoding a RevCAR stimulates the RevCAR and increases the expansion of the RevCAR carrying effector cells and their accumulation at the target site.

In further embodiments, the targeting module is administered simultaneously with the vector or cell comprising a nucleotide sequence encoding a RevCAR.

In further embodiments, the targeting module is administered until, preferably in the range of 3 days to 30 days, after the administration of the vector or cell comprising a nucleotide sequence encoding a RevCAR. Furthermore, additional such doses of the targeting module may be administered following resting periods to reactivate the RevCAR-carrying effector cells.

As used herein, the term "reversible chimeric antigen receptor" refers to an artificial chimeric fusion protein, in particular a receptor comprising a tag, an extracellular hinge and a transmembrane domain and a signal transduction domain. The domains can be derived from different sources and therefore, the receptor is called chimeric. Advantageously, the receptor can bind with the tag to different targeting modules.

Advantageously, the cell comprising a nucleotide sequence encoding a RevCAR expresses the RevCAR, which has binding specificity for the tag-binding domain of the targeting module, which in turn binds to CD123 on a target cell.

In embodiments, the targeting module is in monomeric, dimeric or polymeric form, preferably in monomeric form.

In further embodiments, the targeting module is monovalent, bivalent or multivalent.

In some embodiments, the targeting module according to the invention is bivalent or multivalent and comprises at least one CD123-binding domain and a tag-binding domain that binds to a human La epitope E5B9 comprising a V_{L}-linker-V_{H} structure.

In embodiments, the different domains of the targeting module according to the invention are linked with each other by a linker. The linker comprises a short sequence of preferably 10 to 20 amino acid residues. In embodiments, the targeting module comprises a flexible peptide sequence that is selected such that the domains have a three-dimensional folding that allows them to exhibit the specificity for effector cell and target cell binding. Preferred linkers are glycine-serine linkers with the structure (GₓS_{y}) with x and y selected from 1 to 10, preferably 1 to 5. Mostly preferred are 1 to 10 repeats of the sequence G4S1 (SEQ ID No. 24). Moreover, linkers are preferred that are constituted of a peptide sequence that can increase the protease resistance of the antibody derivatives.

In embodiments, the linker is SEQ ID No. 25 or SEQ ID No. 26.

In embodiments, the targeting module according to the invention comprises a further domain selected from the group comprising co-stimulatory ligands, radionuclides, cell death-inducing chemical compounds and half-life increasing domains, preferably IgG1 Fc, IgG2 Fc, IgG3 Fc, IgG4 F_{c}, HSA, FcRn-binding peptides or mutants thereof. As used herein, the term "mutants" refers to proteins having at least 90 % sequence identity to the half-life increasing domain, preferably at least 95 % sequence identity. Advantageously, the mutant is capable of having one or more activities of the named peptides or proteins; in particular, the mutant increases the half-life like the half-life increasing domain.

In embodiments, the targeting module according to the invention is used in a method for stimulating a chimeric antigen receptor-mediated immune response in a mammal, wherein the targeting module is administered in combination with a vector or a cell comprising a nucleotide sequence encoding a reversible chimeric antigen receptor and at least one further targeting module, wherein the at least one further targeting module comprises at least one target cell-binding domain and a tag-binding domain or a tag, wherein the at least one target cell-binding domain is an antibody, antibody fragment, a protein, a peptide or a low molecular weight organic ligand that binds to surface antigens selected from the group comprising CD2, CD3, CD4, CD8, CD10, CD19, CD20, CD22, CD23, CD25, CD30, CD33, CD38, CD44, CD44v6 CD52, CD90, CD99, CD133, CD150 CD181, CD182, CD184, CD223, CD229, CD269 (BCMA), CD273, CD274, CD276, CD279, CD319, CD366 and CD371, interleukin receptors, especially preferred IL-8Rα (CXCR1), IL-8Rβ (CXCR2), IL-11Rα, IL-11Rβ, IL13Rα1, CXCR4, c-Met, mesothelin, members of the epidermal growth factor receptor family and mutants thereof, especially preferred ErbB1, ErbB2, ErbB3, ErbB4 or mutants thereof, members of the tumor necrosis factor receptor superfamily, ephrins, ephrin receptors, especially preferred EphA1-10, EphA5 or EphB1-6; prostate specific antigens, preferably prostate stem cell antigen (PSCA) and prostate specific membrane antigen (PSMA); embryonic antigen, preferably carcinoembryonic antigen (CEA) and fetal acethylcholine receptor, members of the vascular endothelia growth factor family, epithelia cell adhesion molecule (EpCAM), alphafetoprotein (AFP), members of the intercellular adhesion molecule family, members of the mucin protein family, follicle stimulating hormone receptor (FSHR), the human high molecular weight-melanoma-associated antigen (HMW-MAA), folate binding protein FBP, folate receptors, somatostatin receptors, ligands of the NKG2D receptor, cytokine receptors, members of the epithelia glycoprotein family, diasialogangliosides, glypicans, G protein-coupled receptors, members of the carbonic anhydrase family, members of the carbohydrate antigen family, Notch ligands, melanoma-associated chondroitin sulfate proteoglycan (MCSP), glycoprotein A33, guanylate cyclase 2C and tumor-specific glycans, including mutants and analogues of the named antibodies, antibody fragments, proteins, peptides or low molecular weight organic ligands, wherein the targeting module according to the invention and the at least one further targeting module comprise identical tag-binding domains.

As used herein, the term "target cell-binding domain" refers to a peptide, protein, or low molecular weight organic ligand, which specifically binds a protein or protein complex (antigen) on the surface of a target cell, preferably a cancer cell, T cell, infected cell, pathogens or parasites.

As used herein, the terms "peptide", "polypeptide" and "protein" are used interchangeably, and refer to a compound comprised of amino acid residues covalently linked by peptide bonds. A protein or peptide must contain at least two amino acids, and no limitation is placed on the maximum number of amino acids that can comprise a protein's or peptide's sequence.

As used herein, the term "low molecular weight organic ligand" refers to an organic molecule with a molecular weight of maximal 10 kilodaltons, preferably of maximal 3 kilodaltons, which specifically binds a protein or protein complex (antigen) on the surface of a target cell, preferably a cancer cell, T cell, infected cell or pathogens or parasites.

The term "target cell-binding domain" also comprises soluble T cell receptors, which are composed of the alpha and beta or the gamma and delta chains of a T cell receptor (TCR), fragments or mutants thereof. Such TCR-derived binding moieties recognize and bind to peptides presented by human leukocyte antigen class (HLA) I and II protein complexes. Examples are, but are not limited to, TCRs specific for peptides derived from proteins like EGFR family, survivin, srylike high motility group box (SOX) protein family, melanoma-associated antigens (e.g. autoimmunogenic cancer/testis antigen NY-ESO-1, members of the melanoma antigen family A MAGEA, the preferentially expressed antigen in melanoma PRAME), and leukemia-associated antigens (e.g. Wilms tumor gene 1 WT1).

As used herein, the term "analogues" refers to molecules having a high degree of structural identity to the named antibodies, antibody fragments, proteins, peptides or low molecular weight organic ligands, preferably at least one atom, group of atoms, functional group or substructure is replaced with another group of atoms, e.g. a hydroxy group. In embodiments, an analogue of somatostatin (SRIF14) is octreotide or pasireotide. Advantageously, the analogues bind the identical antigens as the named antibodies, antibody fragments, proteins, peptides or low molecular weight organic ligands.

In embodiments, the analogues of the named antibodies, antibody fragments, proteins or peptides comprise modifications selected from the group comprising D amino acids, pseudo peptide bonds, aminoalcohols, non-proteinogenic amino acids, unnatural amino acids, amino acids with modified side chains and/or circular proteins. Advantageously, these analogues reveal increased stability.

In further embodiments, the target cell-binding domain is a soluble T cell receptor consisting of the alpha and beta or the gamma and delta chain of a T cell receptor (TCR).

In preferred embodiments, the at least one further targeting module comprises at least one target cell-binding domain and a tag-binding domain or a tag, wherein the at least one target cell-binding domain is an antibody or an antibody fragment that binds to surface antigens selected from the group comprising CD2, CD3, CD4, CD8, CD10, CD19, CD20, CD22, CD23, CD25, CD30, CD33, CD38, CD44, CD44v6 CD52, CD90, CD99, CD133, CD150 CD181, CD182, CD184, CD223, CD229, CD269, CD273, CD274, CD276, CD279, CD319, CD366 and CD371, interleukin receptors, especially preferred IL-8Rα, IL-8Rβ, IL-11Rα, IL-11Rβ, IL13Rα1, CXCR4, c-Met, mesothelin, members of the epidermal growth factor receptor family, especially preferred ErbB1, ErbB2, ErbB3 or ErbB4; members of the tumor necrosis factor receptor superfamily, ephrins, ephrin receptors, especially preferred EphA1-10, EphA5 or EphB1-6, prostate specific antigens, preferably PSCA and PSMA; embryonic antigens, preferably CEA and fetal acethylcholine receptor; members of the vascular endothelia growth factor family, EpCAM, AFP, members of the intercellular adhesion molecule family, members of the mucin protein family, FSHR, HMW-MAA, folate binding protein FBP, folate receptors, somatostatin receptors, ligands of the NKG2D receptor, cytokine receptors, members of the epithelia glycoprotein family, diasialogangliosides, glypicans, G protein-coupled receptors, members of the carbonic anhydrase family, members of the carbohydrate antigen family, Notch ligands, MCSP, glycoprotein A33, guanylate cyclase 2C and tumor-specific glycans, wherein the targeting module according to the invention and the at least one further targeting module comprise different target cell-binding domains, and identical tag-binding domains or a tags.

According to the invention, the nucleic acid, vector and/or cell are isolated. As used herein, the term "isolated" means altered or removed from the natural state.

In embodiments, the nucleic acid is a cDNA. As used herein, the term "cDNA" (complementary DNA) refers to double-stranded DNA synthesized from a single-stranded RNA, e.g. mRNA, in a reaction catalyzed by the enzyme reverse transcriptase. In embodiments, cDNA is of synthetic origin. In further embodiments, cDNA is derived from mRNA, therefore containing only exons but no introns, as opposed to genomic DNA.

The vector is preferably a plasmid, an artificial chromosome, linearized DNA or RNA, a virus particle or another vector that contains an expression cassette that is incorporated stably into the genome of a host cell or host organism.

In embodiments, the cell is selected from immune cells, preferably with cytolytic, phagocytic or immunosuppressive activity, such as T cells, Natural Killer (NK) cells and macrophages. In preferred embodiments, the cell is selected from T cells, including alpha/beta and gamma/delta T cells or subpopulations of T cells like stem-cell memory T cells or central memory T cells, cytotoxic T cells or NK cells.

In embodiments, the nucleic acid, vector or cell further comprises an inducible expression system. In some embodiments, the inducible expression system is based on a prokaryotic operon, including, but not limited to, the lac operon, transposon Tn₁₀ or tetracycline operon. In other embodiments, the inducible expression system is based on components of a eukaryotic signaling pathway, including, but not limited to, expression systems based on a steroid receptor, an estrogen receptor, progesterone or metallothionein.

In embodiments, the inducible expression system induces the transcription of the nucleotide sequence encoding a RevCAR and/or a nucleotide sequence encoding a targeting module according to the invention, preferably the inducible expression system induces the transcription of the nucleotide sequence encoding a targeting module according to the invention.

In embodiments, the nucleic acid, vector or cell is administered in combination with a vector or a cell comprising a nucleotide sequence encoding a RevCAR and a nucleic acid, a vector or a cell comprising a nucleotide sequence encoding a further targeting module, wherein the at least one further targeting module comprises at least one target cell-binding domain and a tag-binding domain or a tag, wherein the at least one target cell-binding domain is an antibody, antibody fragment, a protein, a peptide or a low molecular weight organic ligand that binds to surface antigens selected from the group comprising CD2, CD3, CD4, CD5, CD7, CD8, CD10, CD15, CD19, CD20, CD22, CD23, CD25, CD30, CD33, CD38, CD44, CD44v6 CD52, CD66a, CD66b, CD66c, CD66d, CD66e, CD66f, CD90, CD99, CD123, CD133, CD135, CD150 CD181, CD182, CD184, CD223, CD229, CD269, CD273, CD274, CD276, CD279, CD319, CD366 and CD371, cytokine receptors, preferably interleukin receptors, especially preferred IL-8Rα, IL-8Rβ, IL-11Rα, IL-11Rβ, IL13Rα1; CXCR4, c-Met, mesothelin, members of the epidermal growth factor receptor family and mutants thereof, especially preferred ErbB1, ErbB2, ErbB3, ErbB4 or mutants thereof; members of the tumor necrosis factor receptor superfamily, claudins, ephrins, ephrin receptors, especially preferred EphA1-10, EphA5 or EphB1-6; fucosyl transferases, prostate specific antigens, preferably PSCA and PSMA; embryonic antigens, preferably CEA and fetal acethylcholine receptor; members of the vascular endothelia growth factor family, EpCAM, AFP, members of the intercellular adhesion molecule family, C-type lectins, integrins, members of the mucin protein family, FSHR, HMW-MAA, FBP, folate receptors, somatostatin receptors, ligands of the NKG2D receptor, members of the epithelia glycoprotein family, diasialogangliosides, glypicans, G protein-coupled receptors, human papillomavirus proteins, cancer-testis antigens, fibroblast activation proteins, members of the carbonic anhydrase family, members of the carbohydrate antigen family, Notch ligands, MCSP, glycoprotein A33, guanylate cyclase2C and tumor-specific glycans, including mutants and analogues of the named antibodies, antibody fragments, proteins, peptides or low molecular weight organic ligands, wherein the targeting module according to the invention and the at least one further targeting module comprise identical tag-binding domains.

In embodiments, the pharmaceutical composition comprises at least one further targeting module or at least one further nucleic acid, vector or cell encoding a further targeting module, wherein the at least one further targeting module comprises at least one target cell-binding domain and a tag-binding domain or a tag, wherein the at least one target cell-binding domain is an antibody, antibody fragment, a protein, a peptide or a low molecular weight organic ligand that binds to surface antigens selected from the group comprising CD2, CD3, CD4, CD8, CD10, CD19, CD20, CD22, CD23, CD25, CD30, CD33, CD38, CD44, CD44v6 CD52, CD90, CD99, CD133, CD150 CD181, CD182, CD184, CD223, CD229, CD269, CD273, CD274, CD276, CD279, CD319, CD366 and CD371, interleukin receptors, especially preferred IL-8Rα, IL-8Rβ, IL-11Rα, IL-11Rβ, IL13Rα1, CXCR4, c-Met, mesothelin, members of the epidermal growth factor receptor family and mutants thereof, especially preferred ErbB1, ErbB2, ErbB3, ErbB4 or mutants thereof, members of the tumor necrosis factor receptor superfamily, ephrins, ephrin receptors, especially preferred EphA1-10, EphA5 or EphB1-6; prostate specific antigens, preferably PSCA and PSMA; embryonic antigens, preferably CEA and fetal acethylcholine receptor; members of the vascular endothelia growth factor family, EpCAM, AFP, members of the intercellular adhesion molecule family, members of the mucin protein family, FSHR, HMW-MAA, FBP, folate receptors, somatostatin receptors, ligands of the NKG2D receptor, cytokine receptors, members of the epithelia glycoprotein family, diasialogangliosides, glypicans, G protein-coupled receptors, members of the carbonic anhydrase family, members of the carbohydrate antigen family, Notch ligands, MCSP, glycoprotein A33, guanylate cyclase 2C and tumor-specific glycans, including mutants and analogues of the named antibodies, antibody fragments, proteins, peptides or low molecular weight organic ligands, wherein the targeting module and the at least one further targeting module comprise identical tag-binding domains.

According to the invention, the tag-binding domain binds to a tag from the human nuclear La protein. Preferably, the tag-binding domain is an antibody or an antigen-binding fragment, comprising a V_{L} according to the following sequence:
DIVMTQSPDSLAVSLGERATINCX₂₄SSQSLLNSRTX₃₅KNYLAWYQQKPGQPPKLLIYWASTR X₆₁SGVPDRFSGSGSGTDFTLTISSLQAEDVAVYYCKQSYNLX₁₀₁TFGGGTKVEIK (SEQ ID No. 19), wherein X₂₄, X₃₅, X₆₁ and X₁₀₁ are independently from each other selected from a proteinogenic alpha-amino acid residue; or a sequence having at least 90 % sequence identity, preferably at least 95 % sequence identity; to SEQ ID No. 21.

In embodiments, the tag-binding domain constitutes an anti-La epitope scFv.

In some embodiments, X₂₄ to X₁₀₁ are selected as follows:
X₂₄ is selected from polar and/or positive charged residues, such as Serine, Threonine, Asparagine, Glutamine, Histidine, Lysine and Arginine; preferably Lysine or Arginine;
X₃₅ is preferably selected from Lysine and Proline;
X₆₁ is selected from polar and charged residues, such as Asparagine, Aspartic Acid, Glutamine, Glutamic acid, Histidine, Lysine and Arginine, preferably Glutamic acid and Lysine;
X₁₀₁ is selected from hydrophobic residues, such as Isoleucine, Leucine, Valine, Alanine, Methionine, Phenylalanine, Proline and Tryptophan; preferably Leucine or Proline.

Preferably, the tag-binding domain comprises a sequence having each at least 90 % sequence identity, preferably at least 95 % sequence identity; to the sequences according to SEQ ID No. 20 (V_{H}) and SEQ ID No. 21 (V_{L}).

Especially preferred, the tag-binding domain constitutes the anti-La 5B9 scFv according to SEQ ID No. 20 (V_{H}) and SEQ ID No. 21 (V_{L})

As used herein, the term "extracellular hinge and a transmembrane domain" refers to a flexible peptide sequence connected to the tag, which anchors the RevCAR into the cell membrane of the cell and protrudes from the surface of the cell for optimal binding to its particular targeting module.

In embodiments, the extracellular hinge and transmembrane domain are selected from hinge and transmembrane domains of human CD28 molecule, CD8a chain, NK cell receptors, preferably natural killer group NKG2D; or parts of the constant region of an antibody and combinations thereof. As used herein, the term "combinations thereof' refers to combinations of the different hinge and transmembrane domains.

Pinthus et al. and Cartellieri et al. describe the use of hinge and transmembrane domains of the human CD28 molecule in CARs (Pinthus et al. 2003, Cartellieri et al. 2016).

Milone et al. and Zhao et al. describe the use of hinge and transmembrane domains of human CD8a molecule in CARs (Milone et al. 2009, Zhao et al. 2009).

Zhang et al. describe the use of hinge and transmembrane domains of NKG2D in CARs (Zhang et al. 2005).

Frigault et al. and Wang et al. describe the use of hinge and transmembrane domains of parts of the constant region of immunoglobulin G1 (IgG) (Frigault et al. 2015, Wang et al. 2007). Frigault et al. describes the use of hinge domains of the constant region of IgG4.

Examples of combinations of the extracellular hinge and transmembrane domain are, but are not limited to, CD28 extracellular hinge and transmembrane domain, CD8alpha extracellular hinge and transmembrane domain, IgG1 or IgG4 constant regions combined with CD28 or CD137 transmembrane domain.

As used herein, the term "signal transduction domain" refers to a peptide sequence which transmits a signal into the cell by cross-linkage of the cell expressing the RevCAR (effector cell) to a human cell surface protein or protein complex (target cell). Cross-linkage between effector and target cell is mediated by the targeting module according to the invention.

In embodiments, the signal transduction domain is selected from cytoplasmic regions of CD28, CD137 (4-1BB), CD134 (OX40), CD278 (ICOS), DAP10 and CD27, programmed cell death-1 (PD-1), cytotoxic T-lymphocyte antigen 4 (CTLA-4), cytoplasmic regions of CD3 chains, DAP12, CD122 (interleukin-2 receptor β), CD132 (interleukin-2 receptor γ), CD127 (interleukin-7 receptor α), CD360 (interleukin-21 receptor) activating Fc receptors and mutants thereof.

As used herein, the term "mutants" refers to proteins having at least 90 % sequence identity to the signal transduction domains, preferably at least 95 % sequence identity. Advantageously, the mutant transmits a signal into the cell by cross-linkage of the cell expressing the RevCAR (effector cell) to a human cell surface protein or protein complex (target cell) in the same way as the named signal transduction domains.

In embodiments, mutants are truncated versions. As used herein, the term "truncated versions" refers to shortened proteins having at least 90 % sequence identity to the signal transduction domains, preferably at least 95 % sequence identity, more preferably having a chain length of at least 90 % and a sequence identity of 100 %, most preferably a chain length of at least 95 % and a sequence identity of 100 %. Advantageously, the truncated version has an activity of at least 80 %, preferably of at least 90 %, more preferably of at least 95 %; of the named signal transduction domains.

Hombach et al. and Cartellieri et al. describe the use of cytoplasmic regions of CD28 as signal transduction domain in CARs (Hombach et al. 2001, Cartellieri et al. 2016). Guedan et al. describes the use of a mutant of cytoplasmic regions of CD28 as signal transduction domain (Guedan et al. 2020).

Milone et al. and Finney et al. describe the use of cytoplasmic regions of CD137 (4-1BB) as signal transduction domain (Finney et al. 2004, Milone et al. 2009).

Finney et al. and Hombach and Abken describe the use of cytoplasmic regions of CD134 (OX40) as signal transduction domain in CARs (Finney et al. 2004, Hombach and Abken 2011).

Guedan et al. describes the use of cytoplasmic regions of CD278 (ICOS) as signal transduction domain (Guedan et al. 2018).

Zhang et al. describes the use of DAP10 as signal transduction domain (Zhang et al. 2005).

Fedorov et al. describes the use of programmed cell death 1 (PD-1) and of cytotoxic T-lymphocyte antigen 4 (CTLA-4) as signal transduction domain in CARs (Fedorov et al. 2013).

Gong et al. and Gade et al. describe the use of cytoplasmic regions of CD3 chains, in particular the CD3ζ chain, as signal transduction domain in CARs (Gong et al. 1999, Gade et al. 2005).

Töpfer et al. describes the use of DAP12 as signal transduction domain in CARs (Töpfer et al. 2015).

Kagoya et al. describes the use of signaling chains or motifs derived from interleukin receptors as signal transduction domain in CARs (Kagoya et al. 2018).

Lamers et al. and Kershaw et al. describe the use of activating Fc receptors, in particular the Fc epsilon receptor γ chain, as signal transduction domain (Lamers et al. 2004, Kershaw et al*.* 2006).

In preferred embodiments, the signal transduction domain is selected from cytoplasmic regions of CD28, CD137 (4-1BB), CD134 (OX40), CD278 (ICOS), DAP10 and CD27, programmed cell death-1 (PD-1), cytotoxic T-lymphocyte antigen 4 (CTLA-4), cytoplasmic regions of CD3 chains, DAP12, CD122 (interleukin-2 receptor β), CD132 (interleukin-2 receptor γ), CD127 (interleukin-7 receptor α) and CD360 (interleukin-21 receptor) activating Fc receptors.

In further embodiments, the RevCAR comprises a fourth domain, wherein the fourth domain is a short peptide linker in the extracellular portion of the receptor that may serve to detect the chimeric antigen receptor on the cell surface or stimulate the chimeric antigen receptor T cell.

In some embodiments, the fourth domain is located in between the tag-binding domain or the tag and the extracellular hinge domain or an integral part of the extracellular hinge domain.

Advantageously, the RevCAR engrafted cells with the fourth domain can be specifically stimulated to proliferate preferentially and persist longer compared to non-engrafted cells either *in vitro* or *in vivo.* Further advantageously, the fourth domain may be also used to purify RevCAR engrafted cells from mixed cell populations or to dampen RevCAR engrafted cell-mediated immune response and to eliminate RevCAR engrafted cells in vivo.

In further embodiments, the RevCAR comprises a signal peptide. Advantageously, the signal peptide allows for expression on the cell surface of an effector cell. In embodiments, the signal peptide is located at the N-terminus of the RevCAR nucleotide sequence in front of the tag-binding domain or the tag. In some embodiments, the signal peptide targets proteins to the secretory pathway either co-translationally or post-translationally and is selected from leader peptides from proteins like CD28, CD8alpha, IL-2, lysozyme C or the heavy or light chains of antibodies of human origin to avoid immunogenic reactions.

In embodiments, the nucleic acid is a cDNA.

In embodiments, the tag is present at the amino-terminal end of the polypeptide that comprises the RevCAR. Advantageously, locating the tag at the amino terminus permits the tag unhampered access to the targeting module that is bound to the target cell.

In embodiments, the nucleic acid encodes a RevCAR according to SEQ ID No. 29 or SEQ ID No. 30. Preferably, the nucleic acid is SEQ ID No. 31 or SEQ ID No. 32.

In some embodiments, the cell comprising a nucleotide sequence encoding a RevCAR is selected from immune cells, preferably with cytolytic, phagocytic or immunosuppressive activity, such as T cells, Natural Killer (NK) cells and macrophages. In preferred embodiments, the cell is selected from T cells, including alpha/beta and gamma/delta T cells or subpopulations of T cells like stem-cell memory T cells or central memory T cells, cytotoxic T cells; or NK cells.

In embodiments, the kit further comprises at least one further targeting module or at least one further nucleic acid, vector or cell encoding a further targeting module, wherein the at least one further targeting module comprises at least one target cell-binding domain and a tag-binding domain or a tag, wherein the at least one target cell-binding domain is an antibody, antibody fragment, a protein, a peptide or a low molecular weight organic ligand that binds to surface antigens selected from the group comprising CD2, CD3, CD4, CD8, CD10, CD19, CD20, CD22, CD23, CD25, CD30, CD33, CD38, CD44, CD44v6 CD52, CD90, CD99, CD133, CD150 CD181, CD182, CD184, CD223, CD229, CD269, CD273, CD274, CD276, CD279, CD319, CD366 and CD371, interleukin receptors, especially preferred IL-8Rα, IL-8Rβ, IL-11Rα, IL-11Rβ, IL13Rα1, CXCR4, c-Met, mesothelin, members of the epidermal growth factor receptor family and mutants thereof, especially preferred ErbB1, ErbB2, ErbB3, ErbB4 or mutants thereof, members of the tumor necrosis factor receptor superfamily, ephrins, ephrin receptors, especially preferred EphA1-10, EphA5 or EphB1-6, prostate specific antigens, preferably PSCA and PSMA; embryonic antigens, preferably CEA and fetal acethylcholine receptor; members of the vascular endothelia growth factor family, EpCAM, AFP, members of the intercellular adhesion molecule family, members of the mucin protein family, FSHR, HMW-MAA, FBP, folate receptors, somatostatin receptors, ligands of the NKG2D receptor, cytokine receptors, members of the epithelia glycoprotein family, diasialogangliosides, glypicans, G protein-coupled receptors, members of the carbonic anhydrase family, members of the carbohydrate antigen family, Notch ligands, MCSP, glycoprotein A33, guanylate cyclase 2C and tumor-specific glycans, including mutants and analogues of the named antibodies, antibody fragments, proteins, peptides or low molecular weight organic ligands, wherein the targeting module and the at least one further targeting module comprise identical tag-binding domains.

The present invention will now be further explained by the following non-limiting figures and examples.

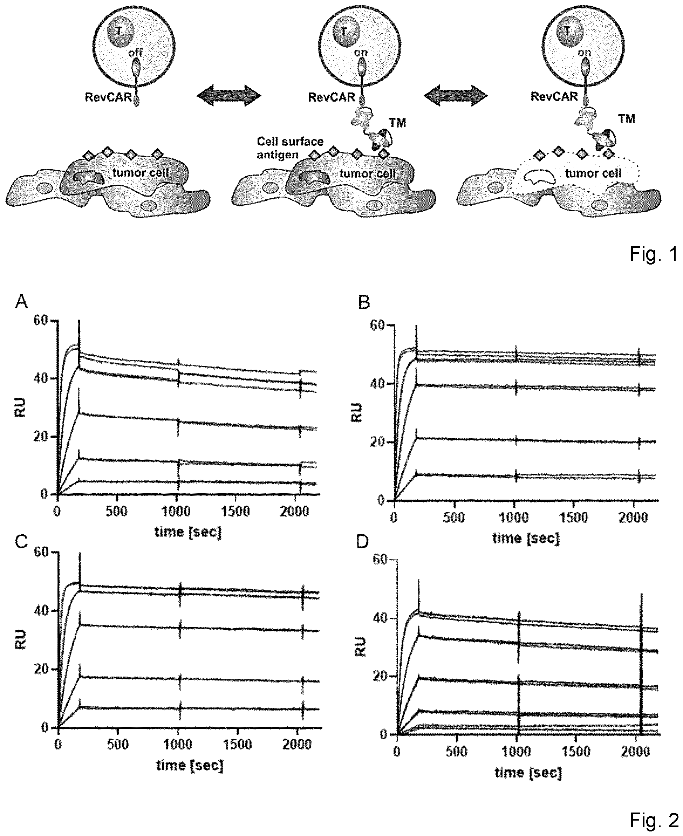

**Fig. 1** shows a schematic illustration of the mode of action of engineered T cell according to the invention in combination with an antigen-specific targeting module (TM) together forming the active drug. A RevCAR expressing allogeneic T cell (Allo-RevCAR-T) carries the RevCAR epitope (RCE or tag), preferably a short, non-immunogenic peptide motif derived from the human nuclear La/SSB autoantigen, on their cell surface. Since ligands binding to RCE or tag are not present within the human body Allo-RevCAR-T remain in an off-mode (left). Antigen-specificity of Allo-RevCAR-T is provided via a soluble targeting module (TM) with exclusive specificity for a target antigen. The TM for Allo-RevCAR consists of a target-binding domain and a tag-binding domain specific for the RCE or tag. Cross-linking of RevCAR-T and the target antigen-expressing tumor cell by TMs activates RevCAR-T effector functions and subsequently killing of the tumor cells (right).
**Fig. 2** shows surface plasmon resonance (SPR) sensograms of targeting modules according to the invention binding to CD123 with a tag-binding domain comprising the structure V_{L}-linker-V_{H} compared to a reference targeting module with the same CD123-binding domain and a tag-binding domain comprising the structure V_{H}-linker-V_{L}: **A)** SEQ ID No. 2 (reference), **B)** SEQ ID No. 5, **C)** SEQ ID No. 6 and **D)** SEQ ID No. 8. For data fitting and K_{D} calculation a 1:1 binding model was applied.

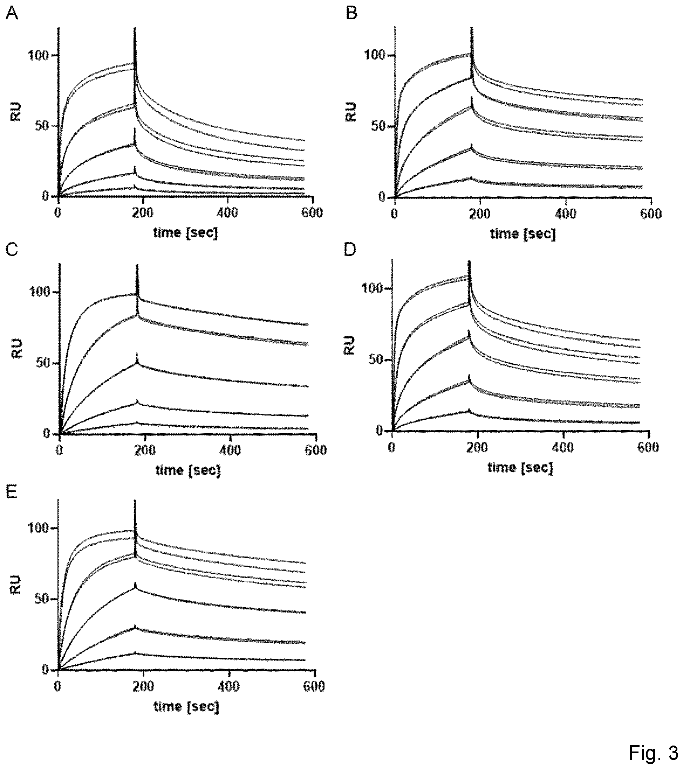

**Fig. 3** shows SPR sensograms of targeting modules according to the invention binding to the La epitope 5B9 according to SEQ ID No. 28 with a tag-binding domain comprising the structure V_{L}-linker-V_{H} compared to a reference targeting module with the same CD123-binding domain and a tag-binding domain comprising the structure V_{H}-linker-V_{L}: **A)** SEQ ID No. 2 (reference), **B)** SEQ ID No. 3, **C)** SEQ ID No. 4, **D)** SEQ ID No. 5 and **E)** SEQ ID No. 6. For data fitting and K_{D} calculation a 1:1 binding model was applied.

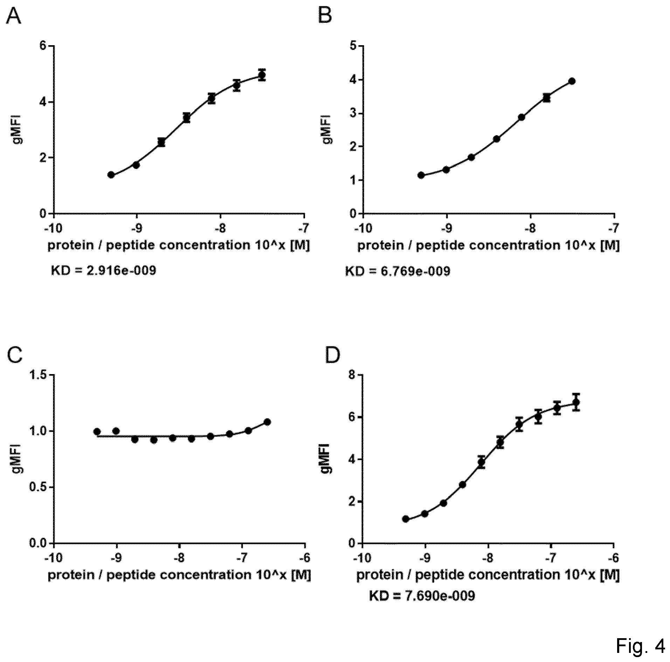

**Fig. 4** shows the cellular binding of targeting modules according to the invention to CD123 positive AML cell lines: **A)** SEQ ID No. 5 to Oci-AML3, **B)** SEQ ID No. 9 to Oci-AML3, **C)** SEQ ID No. 4 to Molm-13 and **D)** SEQ ID No. 6 to Molm-13. The mean fluorescence intensity (MFI) was plotted to obtain the dose-response curve and the half maximal binding (KD) was calculated after fitting with a four logistic regression.

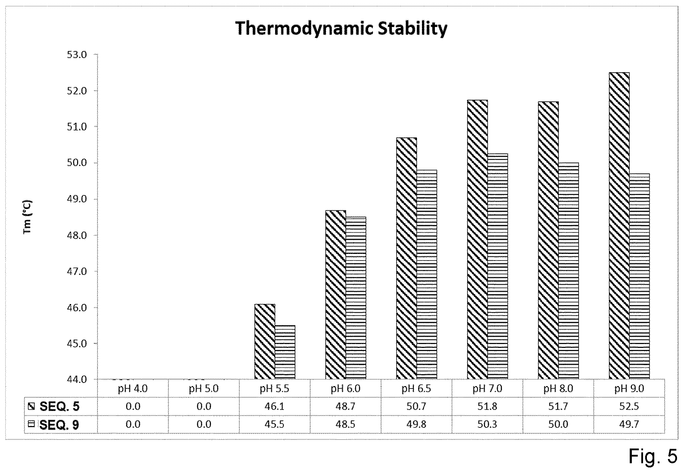

**Fig. 5** shows the thermodynamic stability of two targeting modules according to the invention (SEQ ID No. 5 and SEQ ID No. 9) assessed by melting point analysis in a pH range from pH 4.0 to 9.0.

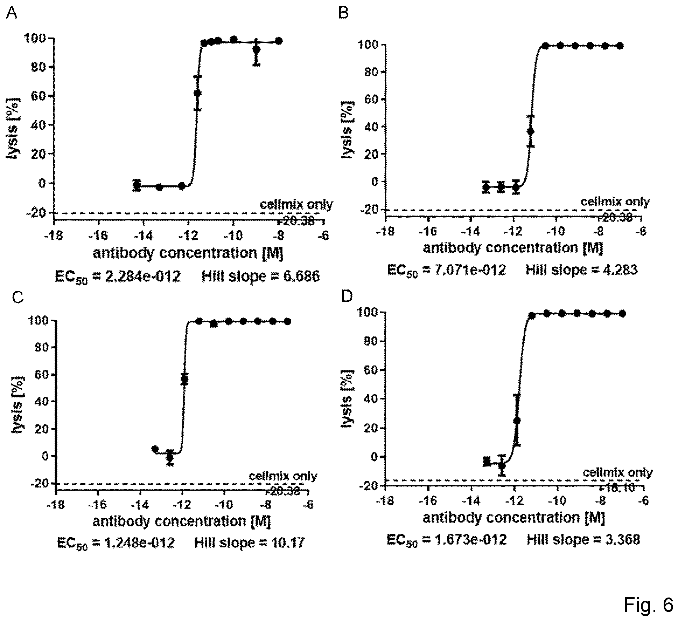

**Fig. 6** shows the results of a cytotoxicity assay of RevCAR T cells and different targeting modules according to the invention against AML cell line Molm-13 using a suspension cell based co-cultivation assay in the presence of variable concentrations of the targeting module: **A)** SEQ ID No. 2 (reference), **B)** SEQ ID No. 4, **C)** SEQ ID No. 5, and **D)** SEQ ID No. 6. The target cell lysis was plotted to obtain a dose-response curve and the EC₅₀ was calculated after fitting with a four logistic regression.

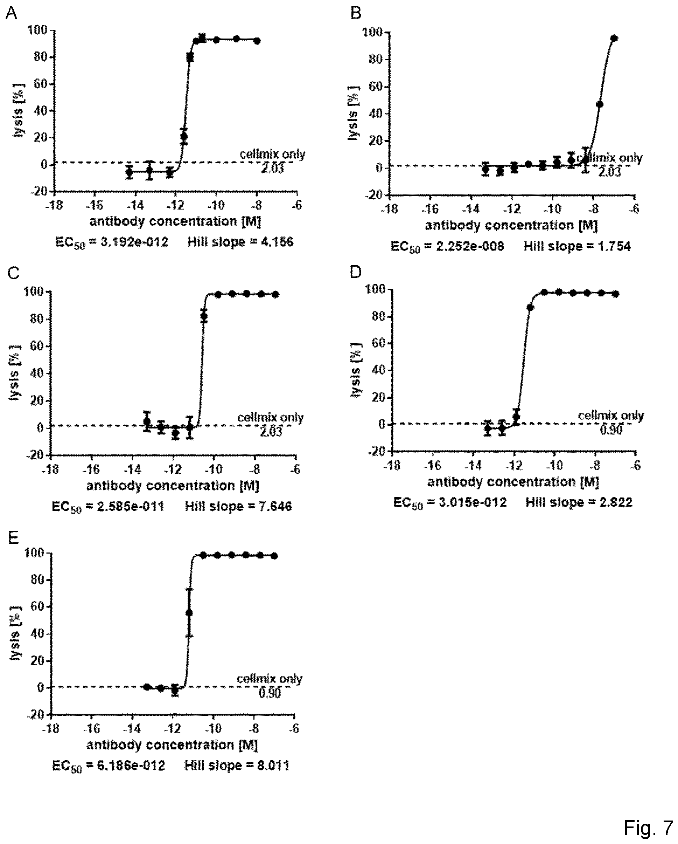

**Fig. 7** shows the results of a cytotoxicity assay of RevCAR T cells and different targeting modules according to the invention against AML cell line line Oci-AML3 using a suspension cell based co-cultivation assay in the presence of variable concentrations of the targeting module: **A)** SEQ ID No. 2 (reference), **B)** SEQ ID No. 3, **C)** SEQ ID No. 4, **D)** SEQ ID No. 5 and **E)** SEQ ID No. 6. The target cell lysis was plotted to obtain a dose-response curve and the EC₅₀ was calculated after fitting with a four logistic regression.

### Design of targeting modules according to the invention

The targeting module R-TM123 is a soluble, recombinant fusion protein comprising two antibody-derived binding domains. One selectively binds to the target antigen CD123, the other recognizes the RCE or tag presented on RevCAR expressing cells (epitope E5B9 from the human La protein). Thus, R-TM123 functions as a bridging module between RevCAR-T and a CD123-expressing target cancer cell (Fig. 1). The targeting module further comprises an 8x-histidine tag for detection and purification purposes at the C-terminus.

**Tab. 1** Design and characteristics of different targeting modules according to the invention (SEQ ID No. 3 to SEQ ID No. 6, SEQ ID No. 8 and SEQ ID No. 9)with a tag-binding domain comprising the structure V_{L}-linker-V_{H} compared to reference targeting modules with the same CD123 binding domain and a tag-binding domain comprising the structure V_{H}-linker-V_{L}: monomer content determined by HPLC, dissociation constants K_{D} determined by SPR and 50% effective concentration (EC₅₀) determined by cellular binding assays (mu = murine, hu = humanized).

| **Targeting module** | **Design** | **Monomer content** | K_{D} **CD123** / **K_{D} 5B9** | **EC₅₀ Oci-AML3** / **EC₅₀ Molm-13** |
|---|---|---|---|---|
| SEQ ID No. 2 (reference) | muCD123 + 5B9-V_{H}-V_{L} | 75% | 227 pM / 17 nM | 3.2 pM / 2.3 pM |
| SEQ ID No. 5 | muCD123 + 5B9-V_{L}-V_{H} | 90% | 23 pM / 3.4 nM | 3.0 pM / 1.2 pM |
| SEQ ID No. 6 | muCD123 + rs5B9-V_{L}-V_{H} | 80% | 44 pM / 2.1 nM | 6.2 pM / 1.7 pM |
| | | | | |
| SEQ ID No. 34 (reference) | huCD123_1 + 5B9-V_{H}-V_{L} | 52% | - | - |
| SEQ ID No. 3 | huCD123_1 + 5B9-V_{L}-V_{H} | 75% | - / 2.0 nM | 23 nM /- |
| SEQ ID No. 4 | huCD123_2+rs5B9-V_{L}V_{H} | 87% | 5.2 nM /3.4 nM | 26 pM / 7.1 pM |
| SEQ ID No. 8 | huCD123_3 + 5B9-V_{L}V_{H} | 94% | 131 pM / - | - |
| SEQ ID No. 9 | huCD123_4 + 5B9-V_{L}V_{H} | 97% | 162 pM / - | - |

### Surface Plasmon Resonance Measurements

The functionality of the CD123-binding TMs can be confirmed in binding assays to soluble recombinant CD123 **(****Fig. 2** **and** **3** and **Tab. 1**) using surface plasmon resonance measurements.

Binding of different variants of the targeting module according to the invention was analyzed by SPR measurements using human CD123-Fc coupled to a CM5 sensor chip on a Biacore X100 device (Fig. 2). Targeting modules were purified monomer except for the targeting module according to SEQ ID No. 8. The targeting modules were concentrated between 1.23 to 100 nM and measured in technical duplicates. For data fitting and K_{D} calculation a 1:1 binding model was applied.

CD123-binding TMs interaction with human CD123 yielded a dissociation constant at equilibrium (K_{D}) in a range between 23 pM und 5.2 nM, while the reference TM according to SEQ ID No. 2 had a K_{D} of 227 pM.

Furthermore, binding of different variants of the targeting module according to the invention was analyzed using the La epitope 5B9 (SEQ ID No. 28) fused to human Fc domain which was coupled to a CM5 sensor chip on a Biacore X100 device (Fig. 3). The targeting modules were purified monomers. The targeting modules were concentrated between 2.47 to 200 nM and measured in technical duplicates. For data fitting and KD calculation a two-state-reaction binding model was applied.

TMs interaction with 5B9 yielded a dissociation constant at equilibrium (K_{D}) in a range between 2.0 nM und 3.4 nM, while the reference TM according to SEQ ID No. 2 had a K_{D} of 17 nM.

### Cellular binding assay

Cellular binding of CD123-binding targeting modules (TM) was tested on CD123 positive target cell lines Oci-AML3 and Molm-13: **A)** SEQ ID No. 5 to Oci-AML3, **B)** SEQ ID No. 9 to Oci-AML3, **C)** SEQ ID No. 4 to Molm-13 and **D)** SEQ ID No. 6 to Molm-13. The affinity of TMs was analyzed by flow cytometry. The TMs were titrated on an AML cell lines and then detected by a mouse anti-His-tag antibody conjugated to phycoerythrin.

Figure 4 shows the cellular binding potential assessed using the CD123 positive target cell lines Oci-AML3 and Molm-13. Different concentrations of R-TM123 were incubated with cells, washed and quantified. A dose-response curve was obtained when the geometric mean fluorescence (MFI) was plotted against the TM concentration.

The data for the cellular binding of CD123-binding TMs on the CD123 positive target cell lines Oci-AML3 and Molm-13 were fitted using a four-parameter model with a variable slope for sigmoidal curves. The 50% effective concentration (EC₅₀) obtained from this model can be interpreted as a representative value of the TM affinity for the cells overexpressing the target receptor (see **Tab. 1**)**.**

### Thermodynamic stability

The thermodynamic stability of two targeting modules according to the invention was assessed by melting point analysis (Fig. 5). A thermal shift assay in different buffers in a pH range from pH 4.0 to 9.0 was used to determine the protein melting point of the targeting module according to SEQ ID No. 5 and SEQ ID No. 9. Typically colloidal stability of proteins correlate with the thermodynamic stability and allows comparative predictability of long term stability.

### RevCAR T cells

For the genetical engineering to express RevCARs, a polynucleotide vector encoding the RevCAR and all necessary elements to ensure its expression in the genetically engineered immune cell is transferred into the immune cell. In particular, the RevCAR comprises IL-2LP (modified human IL-2 leader peptide), RCE (RevCAR epitope, also tag), G4S1 (glycine-serine linker), ECD (extracellular domain), TMD (transmembrane domain), ICD (intracellular domain). The transfer of the vector can be performed by electroporation or transfection of nucleic acids or the help of viral vector systems like adeno-, adeno-associated, retro-, foamy- or lentiviral viral gene transfer.

The lentiviral gene transfer is applied for stable expression of RevCARs in immune cells by first constructing a lentiviral vector encoding for a selected RevCAR. The lentiviral vector is pLVX-EF1alpha UniCAR 28/ζ (Clontech, Takara Bio Group), in which the lentiviral parts of the vector are derived from the human immunodeficiency virus (HIV) and the MSC/IRES/ZxGreenl portion was replaced by the RevCAR construct.

The lentiviral particles are produced by transient transfection of human embryonal kidney (HEK) 293T (ACC 635) cells with the RevCAR encoding lentiviral vector plasmid and cotransfection with a group specific antigen (gag) and Polymerase (pol) encoding plasmid (psPAX2) plus a plasmid encoding for an envelope (pMD2.G). After transfection, the packaging plasmid expresses Gag and Pol protein of HIV-1. The plasmid MD2.G encodes the glycoprotein of the vesicular stomatitis virus (VSV-G). VSV-G protein is used to lentiviral vectors to transduce a broad range of mammalian cells. Various envelopes from different virus species can be utilized for this purpose. Lentiviral vectors can successfully pseudotype with the envelope glycoproteins (Env) of amphotropic murine leukemia virus (MLV) or the G protein of vesicular stomatitis virus (VSV-G), a modified envelope of the prototypic foamy virus (PFV) or chimeric envelope glycoprotein variants derived from gibbon ape leukemia virus (GaLV) and MLV.

Supernatants from transfected HEK293T cells are harvested 24 h to 96 h after transfection and virus particles are concentrated from the supernatant by ultracentrifugation or other methods. For lentiviral transduction of immune cells, *peripheral blood mononuclear cells* (PBMC) or isolated T cells are activated with mab specific for the CD3 complex, e.g. clone OKT3 or UCHT1, either given in solution or coated to plastic cell culture dishes or magnetic beads or a biodegradable polymer matrix. Activation of PBMC or isolated T cells is further enhanced by stimulating costimulatory pathways with mabs or ligands specific for CD27, CD28, CD134 or CD137 either alone or in combinations coated to plastic cell culture dishes or magnetic beads or a biodegradable polymer matrix and the supply with exogenous recombinant cytokines like interleukin (IL)-2, IL-7, IL-12, IL-15 and IL-21. Concentrated or non-concentrated virus particles are added to PBMC or T cell cultures 24 h to 96 h after initial administration of activating CD3 specific antibodies and/or antibodies specific for costimulatory receptors CD27, CD28, CD134 or CD137 and/or recombinant cytokines as single or multiple doses. T cell electroporation, transduction and expansion may be performed in open cell culture systems by manual handling or in closed partially or fully automated systems.

Stable transduction of T cells may be determined by flow cytometry after staining with tag-containing molecules for surface expression of RevCARs or mabs directed against a fourth domain of RevCARs from day 3 onwards after the final administration of virus supernatant. RevCAR transduced T cells can be propagated *in vitro* by culturing them under the supply of recombinant cytokines and activating anti-CD3 mabs.

In case the RevCAR harbors the optional fourth domain, a peptide sequence forming a linear epitope for a mab, immune cells genetically modified to express RevCARs can be specifically propagated *in vitro* by coating a mab or antibody fragments thereof binding to the fourth RevCAR domain to the surface of culture dishes or to beads of any kind or a biodegradable polymer matrix, which are added to the cell culture at a defined ratio. The binding of surface-coated mabs to the RevCAR peptide domain induces cross-linkage of cell-surface expressed RevCARs and formation of an immune synapse, which leads to the activation of signal pathways specifically triggered by the signal domain of the RevCAR. Depending on the signal pathways induced, this may lead to enhance proliferation and sustained resistance against activation-induced cell death of the RevCAR-carrying immune cells and therefore enrichment of RevCAR genetically modified immune cells in a mixed population.

The optional fourth domain, a peptide sequence forming a linear epitope for a mab, can be further utilized to enrich and purify RevCAR-expressing immune cells from mixed populations. Enrichment and purification are performed with the help of a mab or antibody fragment thereof binding to the fourth RevCAR domain to either mark RevCAR-expressing cells for cell sorting or to transiently link the RevCAR expressing immune cell to small particles, which can be utilized for cell isolation. In one aspect, RevCAR-engrafted immune cells are incubated with the mab recognizing the fourth domain. Next, magnetic beads are added, which are conjugated with antibodies or fragments thereof directed against the species- and isotype-specific heavy and light chains of the mab binding to the optional fourth domain. Thus, RevCAR-expressing immune cells and magnetic beads are linked and are trapped and separated from other immune cells in a magnetic field.

### Cytotoxicity assay

The potency of CD123-binding TMs to induce a tumor cell elimination by RevCAR-T cells was tested using a suspension cell based co-cultivation assay with the AML cell line Molm-13 **(****Fig. 6****)** and the AML cell line Oci-AML3 **(****Fig. 7****)** in the presence of variable concentrations of the targeting module. Switchable CAR-T cells were incubated with the target cells at a E:T ratio of 2:1 in the presence of various TM concentrations for 48 h. As CD123-positive target cells the human AML cell line Molm-13 **(****Fig. 6****)** or Oci-AML3 **(****Fig. 7****),** respectively, was used which was stained with efluor prior setup. Target cells were quantified by flow cytometry and lysis was calculated normalizing the cell count of each sample to a control sample where only tumor cells were plated. Data were fitted with a four-parameter model with a variable slope for sigmoidal curves. The calculated EC₅₀ value can be interpreted as a representative value for the TM potency against these tumor cells.

### Cited non-patent literature

Brudno JN, Kochenderfer JN (2016) Toxicities of chimeric antigen receptor T cells: recognition and management. Blood 127, 3321-3330.
Cartellieri M, Bachmann M, Feldmann A, Bippes C, Stamova S, Wehner R, Temme A, Schmitz M (2010) Chimeric Antigen Receptor-Engineered T Cells for Immunotherapy of Cancer J. Biomed. Biotechnol. Article ID 956304, doi: 10.1155/2010/956304.
Cartellieri M, Feldmann A, Koristka S, Arndt C, Loff S, Ehninger A, von Bonin M, Bejestani EP, Ehninger G, Bachmann MP (2016) Switching CAR T cells on and off: a novel modular platform for retargeting of T cells to AML blasts. Blood cancer J. 6 (8), e458.
Fedorov VD, Themeli M, Sadelain M (2013) PD-1- and CTLA-4-based inhibitory chimeric antigen receptors (iCARs) divert off-target immunotherapy responses. Sci. Transl. Med. 5 (215), 215ra172.
Finney HM, Akbar AN, Lawson AD (2004) Activation of resting human primary T cells with chimeric receptors: costimulation from CD28, inducible costimulator, CD134, and CD137 in series with signals from the TCR zeta chain. J. Immunol. 172 (1), 104-113.
Frigault MJ, Lee J, Basil MC, Carpenito C, Motohashi S, Scholler J, Kawalekar OU, Guedan S, McGettigan SE, Posey AD Jr, Ang S, Cooper LJ, Platt JM, Johnson FB, Paulos CM, Zhao Y, Kalos M, Milone MC, June CH (2015) Identification of chimeric antigen receptors that mediate constitutive or inducible proliferation of T cells. Cancer Immunol. Res. 3 (4), 356-367.
Gade TP, Hassen W, Santos E, Gunset G, Saudemont A, Gong MC, Brentjens R, Zhong XS, Stephan M, Stefanski J, Lyddane C, Osborne JR, Buchanan IM, Hall SJ, Heston WD, Rivière I, Larson SM, Koutcher JA, Sadelain M (2005) Targeted elimination of prostate cancer by genetically directed human T lymphocytes. Cancer Res. 65 (19), 9080-9088.
Gong MC, Latouche JB, Krause A, Heston WDW, Bander NH, Sadelain M (1999) Cancer patient T cells genetically targeted to prostate-specific membrane antigen specifically lyse prostate cancer cells and release cytokines in response to prostate-specific membrane antigen. Neoplasia. 1 (2), 123-127.
Guedan S, Posey AD, Shaw C, Wing A, Da T, Patel PR, McGettigan SE, Casado-Medrano V, Kawalekar OU, Uribe-Herranz M, Song D, Melenhorst JJ, Lacey SF, Scholler J, Keith B, Young RM, June CH (2018) Enhancing CAR T cell persistence through ICOS and 4-1BB costimulation. JCI Insight. 3(1), 96976.
Guedan S, Madar A, Casado-Medrano V, Shaw CE, Wing A, Liu F, Young RM, June CH, Posey AD (2020) Single residue in CD28-costimulated CAR T cells limits long-term persistence and antitumor durability. J Clin Invest. 133215.
Hombach AA, Abken H (2011) Costimulation by chimeric antigen receptors revisited the T cell antitumor response benefits from combined CD28-OX40 signalling. Int. J. Cancer. 129 (12), 2935-2944.
Hombach A, Sent D, Schneider C, Heuser C, Koch D, Pohl C, Seliger B, Abken H (2001) T-cell activation by recombinant receptors: CD28 costimulation is required for interleukin 2 secretion and receptor-mediated T-cell proliferation but does not affect receptor-mediated target cell lysis. Cancer Res. 61 (5), 1976-1982.
Kagoya Y, Tanaka S, Guo T, Anczurowski M, Wang CH, Saso K, Butler MO, Minden MD, Hirano N (2018) A novel chimeric antigen receptor containing a JAK-STAT signaling domain mediates superior antitumor effects. Nat Med. 24(3), 352-359.
Kershaw MH, Westwood JA, Parker LL, Wang G, Eshhar Z, Mavroukakis SA, White DE, Wunderlich JR, Canevari S, Rogers-Freezer L, Chen CC, Yang JC, Rosenberg SA, Hwu P (2006) A phase I study on adoptive immunotherapy using gene-modified T cells for ovarian cancer. Clin. Cancer Res. 12 (20), 6106-6115.
Lamers CH, Sleijfer S, Willemsen RA, Debets R, Kruit WHJ, Gratama JW, Stoter G (2004) Adoptive immuno-gene therapy of cancer with single chain antibody [scFv(lg)] gene modified T lymphocytes. J. Biol. Regul. Homeost. Agents. 18 (2), 134-140.
Milone MC, Fish JD, Carpenito C, Carroll RG, Binder GK, Teachey D, Samanta M, Lakhal M, Gloss B, Danet-Desnoyers G, Campana D, Riley JL, Grupp SA, June CH (2009) Chimeric receptors containing CD137 signal transduction domains mediate enhanced survival of T cells and increased antileukemic efficacy in vivo. Mol. Ther. 17 (8), 1453-1464.
Morgan RA, Yang JC, Kitano M, Dudley ME, Laurencot CM, Rosenberg SA (2010) Case Report of a Serious Adverse Event Following the Administration of T Cells Transduced With a Chimeric Antigen Receptor Recognizing ERBB2. Mol. Ther. 18, 843-851.
Pinthus JH, Waks T, Kaufman-Francis K, Schindler DG, Harmelin A, Kanety H, Ramon J, Eshhar Z (2003) Immuno-gene therapy of established prostate tumors using chimeric receptor-redirected human lymphocytes. Cancer Res. 63 (10), 2470-2476.
Sotillo E, Barrett DM, Black K., Bagashev A, Oldridge D, Wu G, Sussman R, Lanauze C, Ruella M, Gazzara MR, Martinez NM, Harrington CT, Chung EY, Perazzelli J, Hofmann TJ, Maude SL, Raman P, Barrera A, Gill S, Lacey SF, Melenhorst JJ, Allman D, Jacoby E, Fry T, Mackall C, Barash Y, Lynch KW, Maris JM, Grupp SA, Thomas-Tikhonenko A (2015) Convergence of Acquired Mutations and Alternative Splicing of CD19 Enables Resistance to CART-19 Immunotherapy. Cancer Discov. 5, 1282-1295.
Titov A, Petukhov A, Staliarova A, Motorin D, Bulatov E, Shuvalov O, Soond SM, Piacentini M, Melino G, Zaritskey A, Barlev NA (2018) The biological basis and clinical symptoms of CAR-T therapy-associated toxicites. Cell Death Dis 9, 897.
Töpfer K, Cartellieri M, Michen S, Wiedemuth R, Müller N, Lindemann D, Bachmann M, Füssel M, Schackert G, Temme A (2015) DAP12-based activating chimeric antigen receptor for NK cell tumor immunotherapy. J. Immunol. 194 (7), 3201-3212.
Wang J, Jensen M, Lin Y, Sui X, Chen E, Lindgren CG, Till B, Raubitschek A, Forman SJ, Qian X, James S, Greenberg P, Riddell S, Press OW (2007) Optimizing adoptive polyclonal T cell immunotherapy of lymphomas, using a chimeric T cell receptor possessing CD28 and CD137 costimulatory domains. Hum. Gene Ther. 18 (8), 712-725.
Zhang T, Lemoi BA, Sentman CL (2005) Chimeric NK-receptor-bearing T cells mediate antitumor immunotherapy. Blood. 106 (5), 1544-1551.
Zhao Y, Wang QJ, Yang S, Kochenderfer JN, Zheng Z, Zhong X, Sadelain M, Eshhar Z, Rosenberg SA, Morgan RA (2009) A herceptin-based chimeric antigen receptor with modified signaling domains leads to enhanced survival of transduced T lymphocytes and antitumor activity. J. Immunol. 183 (9), 5563-5574.

## Claims

1. A targeting module comprising
i) at least one CD123-binding domain comprising a sequence with at least 95 % identity, preferably 99 % identity, with the sequence according to SEQ ID No. 22 and SEQ ID No. 23, and
ii) a tag-binding domain binding a human La epitope E5B9 comprising a V_{L}-linker-V_{H} structure, wherein the V_{L} region of the tag-binding domain comprises a sequence with at least 95 % identity, preferably 99 % identity, with the sequence according to SEQ ID No. 19 and/or the V_{H} region of the tag-binding domain comprises a sequence with at least 95 % identity, preferably 99 % identity, with the sequence according to SEQ ID No. 20.

2. The targeting module according to claim 1, wherein the linker of the tag-binding domain comprises 20 to 30 amino acids, preferably 25 amino acids.

3. The targeting module according to claim 1 or 2, wherein the linker of the tag-binding domain comprises a linker according to one of the sequences SEQ ID No. 25 or SEQ ID No. 26.

4. The targeting module according to one of the claims 1 to 3, wherein the CD123-binding domain is an antibody or antigen binding fragment.

5. The targeting module according to one of the claims 1 to 4, wherein the CD123-binding domain comprises a sequence according to SEQ ID No. 27.

6. The targeting module according to one of the claims 1 to 5, wherein the length is in the range of 20 to 1600 amino acids, preferably 200 to 800 amino acids.

7. The targeting module according to one of the claims 1 to 6 comprising one of the sequences SEQ ID No. 3 to SEQ ID No. 10.

8. A nucleic acid, a vector or a cell comprising a nucleotide sequence encoding a targeting module according to one of the claims 1 to 7.

9. The nucleic acid, vector or cell according to claim 8 comprising one of the sequences SEQ ID No. 11 to SEQ ID No. 18.

10. A pharmaceutical composition comprising the targeting module according to one of the claims 1 to 7 and a pharmaceutically acceptable thinner or carrier.

11. The targeting module according to one of the claims 1 to 7, a nucleic acid, a vector or a cell comprising a nucleotide sequence encoding the targeting module according to one of the claims 1 to 7 or a pharmaceutical composition comprising the targeting module according to one of the claims 1 to 7 and a pharmaceutically acceptable thinner or carrier for use in a method for stimulating a chimeric antigen receptor-mediated immune response in a mammal, preferably for use in the treatment of cancer, infectious disease or autoimmune disease.

12. The targeting module for use in a method for stimulating a chimeric antigen receptor-mediated immune response in a mammal according to claim 11,
wherein the targeting module is administered in combination with a vector or a cell comprising a nucleotide sequence encoding a reversible chimeric antigen receptor, wherein the reversible chimeric antigen receptor comprises
- a tag, wherein the tag is the human La epitope E5B9,
- an extracellular hinge and transmembrane domain and
- a signal transduction domain,
wherein the tag-binding domain of the targeting module binds to the tag of the reversible chimeric antigen receptor.

13. The pharmaceutical composition for use in a method for stimulating a chimeric antigen receptor-mediated immune response in a mammal according to claim 11 further comprising a vector or a cell comprising a nucleotide sequence encoding a reversible chimeric antigen receptor, wherein the reversible chimeric antigen receptor comprises
- a tag, wherein the tag is the human La epitope E5B9,
- an extracellular hinge and transmembrane domain and
- a signal transduction domain,
wherein the tag-binding domain of the targeting module binds to the tag of the reversible chimeric antigen receptor.

14. A kit comprising
a) a targeting module according to one of the claims 1 to 7 or the nucleic acid, vector or cell according to claim 8 or 9, and
b) a vector or a cell comprising a nucleotide sequence encoding a reversible chimeric antigen receptor,
wherein the reversible chimeric antigen receptor comprises
- a tag, wherein the tag is the human La epitope E5B9,
- an extracellular hinge and transmembrane domain and
- a signal transduction domain,
wherein the tag-binding domain of the targeting module binds to the tag of the
reversible chimeric antigen receptor.

15. The kit according to claim 14, wherein the extracellular hinge and transmembrane domain is selected from the group comprising a hinge and transmembrane domain of a human CD28 molecule, a CD8a chain NK cell receptor, parts of the constant region of an antibody and combinations thereof.

16. The kit according to claim 14 or 15, wherein the signal transduction domain is selected from the group comprising cytoplasmic regions of CD28, CD137 (4-1BB), CD134 (OX40), CD278 (ICOS), DAP10 and CD27, programmed cell death-1 (PD-1), cytotoxic T-lymphocyte antigen 4 (CTLA-4), cytoplasmic regions of CD3 chains, DAP12, CD122 (interleukin-2 receptor β), CD132 (interleukin-2 receptor γ), CD127 (interleukin-7 receptor α), CD360 (interleukin-21 receptor), activating Fc receptors and mutants thereof.

17. The kit according to one of the claims 14 to 16 further comprising at least one further targeting module or at least one further nucleic acid, vector or cell encoding a further targeting module, wherein the at least one further targeting module comprises at least one target cell-binding domain and a tag-binding domain,
wherein the at least one further target cell-binding domain is an antibody, antibody fragment, a protein, a peptide or a low molecular weight organic ligand that binds to surface antigens selected from the group comprising CD2, CD3, CD4, CD5, CD7, CD8, CD10, CD15, CD19, CD20, CD22, CD23, CD25, CD30, CD33, CD38, CD44, CD44v6 CD52, CD66a, CD66b, CD66c, CD66d, CD66e, CD66f, CD90, CD99, CD133, CD135, CD150 CD181, CD182, CD184, CD223, CD229, CD269, CD273, CD274, CD276, CD279, CD319, CD366 and CD371, cytokine receptors, CXCR4, c-Met, mesothelin, members of the epidermal growth factor receptor family and mutants thereof, members of the tumor necrosis factor receptor superfamily, claudins, ephrins, ephrin receptors, fucosyl transferases, prostate specific antigens, embryonic antigens, members of the vascular endothelia growth factor family, EpCAM, AFP, members of the intercellular adhesion molecule family, C-type lectins, integrins, members of the mucin protein family, FSHR, HMW-MAA, FBP, folate receptors, somatostatin receptors, ligands of the NKG2D receptor, members of the epithelia glycoprotein family, diasialogangliosides, glypicans, G protein-coupled receptors, human papillomavirus proteins, cancer-testis antigens, fibroblast activation proteins, members of the carbonic anhydrase family, members of the carbohydrate antigen family, Notch ligands, MCSP, glycoprotein A33, guanylate cyclase 2C and tumor-specific glycans,
wherein the targeting module and the at least one further targeting module comprise identical tag-binding domains.

18. The kit according to one of the claims 14 to 17, wherein the targeting module and/or the vector or cell comprising a nucleotide sequence encoding a reversible chimeric antigen receptor are in the form of a pharmaceutical composition.

19. The kit according to one of the claims 14 to 18 for use in a method for stimulating a chimeric antigen receptor-mediated immune response in a mammal, preferably for use in the treatment of cancer, infectious disease or autoimmune disease.
